# EUROPEAN PATENT APPLICATION

(11) **EP 4 851 588 A1**
(43) Date of publication of application: **09.12.2026**
(21) Application number: 25180419.1
(22) Date of filing: 03.06.2025
(51) Int. Cl.: G01J 3/02, G01J 3/44, G01J 3/28

(54) **APPARATUS AND METHOD FOR RAMAN SPECTROSCOPY**

(71) Applicant: Infineon Technologies AG, 85579 Neubiberg (DE)
(72) Inventor: LEITNER, Raimund, 9500 Villach (AT); SCHICKBICHLER, Wolfgang, 8605 Kapfenberg (AT); BERGER, Armin, 8041 Graz (AT); BESHINSKI, Krum, 8101 Gratkorn (AT)
(74) Representative: 2SPL Patentanwälte PartG mbB

(57) **Abstract**

Provided is an apparatus for Raman spectroscopy. The apparatus includes an illumination source configured to emit, for a plurality of measurement cycles in a measurement frame, respective excitation light to induce Raman scattering in a sample. In addition, the apparatus includes a pixel arrangement. The pixel arrangement includes at least one photo-sensitive pixel. The pixel arrangement further includes a first charge storage and a second charge storage having respective charge levels configured to vary in response to interactions with charge carriers generated by the pixel arrangement. The charge carriers are controlled by the pixel arrangement to interact with the charge storages in response to the excitation light based on at least one control signal. The at least one control signal is configured to control the pixel arrangement in each measurement cycle to a) cause the charge carriers to vary the charge level of the first charge storage during an initial first phase of the respective measurement cycle for measuring background light; b) cause the charge carriers to vary the charge level of the second charge storage during a second phase of the respective measurement cycle following the first phase for measuring Raman light generated in response to the excitation light, the excitation light being emitted during the second phase of the respective measurement cycle; and c) drain the charge carriers from the at least one photo-sensitive pixel during a third phase of the respective measurement cycle extending from the end of the second phase through to the end of the respective measurement cycle. The pixel arrangement is configured to prevent interactions of the first and second charge storages with the charge carriers drained from the at least one photo-sensitive pixel during the third phase. An output of the pixel arrangement for the measurement frame is based on a voltage difference between the first and the second charge storage at the end of the measurement frame.

## Description

### Field

The present disclosure relates to Raman spectroscopy. In particular, examples of the present disclosure relate to an apparatus and a method for Raman spectroscopy.

### Background

Raman spectroscopy is a widely used technique for identifying molecular compositions based on inelastic scattering of light. It finds applications in diverse fields, including medical diagnostics, food quality analysis, and industrial monitoring. However, practical implementation of Raman spectroscopy faces challenges due to the inherently weak Raman signals, which are obscured by autofluorescence and ambient light. Conventional approaches typically rely on expensive and complex components, such as scientific-grade cameras or advanced filters, which are unsuitable for mass-market devices.

Hence, there may be a desire for improved Raman spectroscopy.

### Summary

This demand is met by the subject-matter of the independent claims. Further embodiments are addressed by the dependent claims.

According to a first aspect, the present disclosure provides an apparatus for Raman spectroscopy. The apparatus comprises an illumination source configured to emit, for a plurality of measurement cycles in a measurement frame, respective excitation light to induce Raman scattering in a sample. In addition, the apparatus comprises a pixel arrangement. The pixel arrangement comprises at least one photo-sensitive pixel. The pixel arrangement further comprises a first charge storage and a second charge storage having respective charge levels configured to vary in response to interactions with charge carriers generated by the pixel arrangement. The charge carriers are controlled by the pixel arrangement to interact with the charge storages in response to the excitation light based on at least one control signal. The at least one control signal is configured to control the pixel arrangement in each measurement cycle to a) cause the charge carriers to vary the charge level of the first charge storage during an initial first phase of the respective measurement cycle for measuring background light; b) cause the charge carriers to vary the charge level of the second charge storage during a second phase of the respective measurement cycle following the first phase for measuring Raman light generated in response to the excitation light, the excitation light being emitted during the second phase of the respective measurement cycle; and c) drain the charge carriers from the at least one photo-sensitive pixel during a third phase of the respective measurement cycle extending from the end of the second phase through to the end of the respective measurement cycle. The pixel arrangement is configured to prevent interactions of the first and second charge storages with the charge carriers drained from the at least one photo-sensitive pixel during the third phase. An output of the pixel arrangement for the measurement frame is based on a voltage difference between the first and the second charge storage at the end of the measurement frame.

According to a second aspect, the present disclosure provides a handheld or wearable device comprising an apparatus for Raman spectroscopy according to the first aspect.

According to a third aspect, the present disclosure provides a method for Raman spectroscopy using a pixel arrangement. The pixel arrangement comprises at least one photo-sensitive pixel. The pixel arrangement further comprises a first charge storage and a second charge storage having respective charge levels configured to vary in response to interactions with charge carriers generated by the pixel arrangement. The method comprises emitting, for a plurality of measurement cycles in a measurement frame, respective excitation light to induce Raman scattering in a sample. The charge carriers are controlled by the pixel arrangement to interact with the charge storages in response to the excitation light based on at least one control signal. The method further comprises controlling the pixel arrangement via the at least one control signal in each measurement cycle to a) cause the charge carriers to vary the charge level of the first charge storage during an initial first phase of the respective measurement cycle for measuring background light; b) cause the charge carriers to vary the charge level of the second charge storage during a second phase of the respective measurement cycle following the first phase for measuring Raman light generated in response to the excitation light, the excitation light being emitted during the second phase of the respective measurement cycle; and c) drain the charge carriers from the at least one photo-sensitive pixel during a third phase of the respective measurement cycle extending from the end of the second phase through to the end of the respective measurement cycle. The pixel arrangement prevents interactions of the first and second charge storages with the charge carriers drained from the at least one photo-sensitive pixel during the third phase. The method comprises providing an output of the pixel arrangement for the measurement frame based on a voltage difference between the first and the second charge storage at the end of the measurement frame.

According to a fourth aspect, the present disclosure provides use of the apparatus according to the first aspect, the handheld or wearable device according to the second aspect, or the method according to the third aspect for one or more of determining a physiological property of an organism and foodstuff analysis.

### Brief description of the Figures

Some examples of apparatuses and/or methods will be described in the following by way of example only, and with reference to the accompanying figures, in which
Fig. 1 schematically illustrates an example of an apparatus for Raman spectroscopy;
Fig. 2 illustrates an exemplary composition of incident light;
Fig. 3 illustrates an exemplary timing diagram;
Fig. 4 schematically illustrates an exemplary handheld or wearable device; and
Fig. 5 illustrates a flowchart of an example of a method for Raman spectroscopy.

### Detailed Description

Some examples are now described in more detail with reference to the enclosed figures. However, other possible examples are not limited to the features of these embodiments described in detail. Other examples may include modifications of the features as well as equivalents and alternatives to the features. Furthermore, the terminology used herein to describe certain examples should not be restrictive of further possible examples.

Throughout the description of the figures same or similar reference numerals refer to same or similar elements and/or features, which may be identical or implemented in a modified form while providing the same or a similar function. The thickness of lines, layers and/or areas in the figures may also be exaggerated for clarification.

When two elements A and B are combined using an "or", this is to be understood as disclosing all possible combinations, i.e., only A, only B as well as A and B, unless expressly defined otherwise in the individual case. As an alternative wording for the same combinations, "at least one of A or B" or "A and/or B" may be used. This applies equivalently to combinations of more than two elements.

If a singular form, such as "a", "an" and "the" is used and the use of only a single element is not defined as mandatory either explicitly or implicitly, further examples may also use several elements to implement the same function. If a function is described below as implemented using multiple elements, further examples may implement the same function using a single element or a single processing entity. It is further understood that the terms "include", "including", "comprise" and/or "comprising", when used, describe the presence of the specified features, integers, steps, operations, processes, elements, components and/or a group thereof, but do not exclude the presence or addition of one or more other features, integers, steps, operations, processes, elements, components and/or a group thereof.

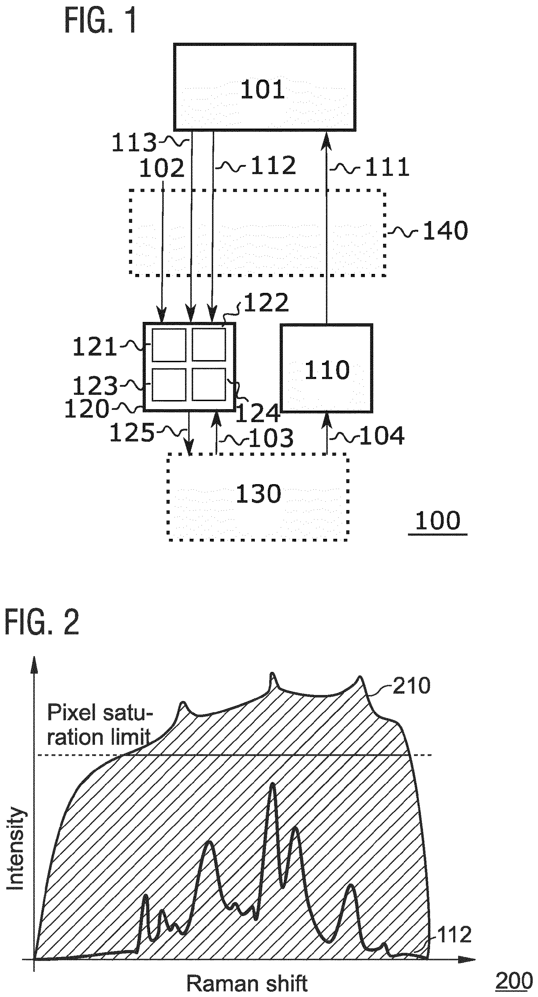

**Fig. 1** schematically illustrates an apparatus 100 for Raman spectroscopy.

The apparatus 100 comprises an illumination source (light source, light emitting component) 110. The illumination source 110 may, e.g., comprise or be one or more Light-Emitting Diode (LED) or laser diode such as a Vertical-Cavity Surface-Emitting Laser (VCSEL) or an Edge-Emitting Laser (EEL). A VCSEL may be used for the illumination source 110 due to its compact size, high efficiency, and directed light output. However, it should be noted that the present disclosure is not limited thereto. Other types of light sources may be used as well.

The illumination source 110 is configured to emit, for a plurality of measurement cycles in a (first) measurement frame, a respective excitation light 111 to induce Raman scattering in a sample 101. The respective excitation light 111 may be a respective excitation light pulse. For example, the emission of the excitation light 111 may be driven based on a drive signal 104. The illumination source 110 may either directly receive the drive signal 104 and control emission of the excitation light 111 based thereon, or intermediate drive circuitry (not illustrated) may receive the drive signal 104 and drive the light source 110 based thereon.

The excitation light 111 is (e.g., pulsed) light suitable to interact with the molecular structure of the sample 101. The excitation light 111 may, e.g., exhibit a wavelength in the visible or near-infrared range (e.g., a wavelength between 400 nm and 1100 nm).

The sample 101 is a material or object subjected to Raman spectroscopy by the apparatus 100. The sample 101 may, e.g., include biological materials, such as foodstuff, tissues or fluids from an organism, or non-biological materials, such as beverages, foodstuff or chemical substances, whose characteristics are to be analyzed based on Raman scattering.

Raman scattering is a process in which incident photons of the excitation light 111 interact with the molecules of the sample 101 and are scattered inelastically with a change in energy. The energy difference between the incident and scattered photons corresponds to the vibrational, rotational, or other low-frequency modes of the molecules. This energy difference is known as the Raman shift. The specific shifts provide a characteristic Raman signal (Raman light) 112, which is a unique spectral fingerprint of the sample 101, that can be used to identify the molecular structure and/or chemical composition of the sample 101.

The measurement frame is a discrete time interval in which the apparatus 100 performs light emission and sensing for Raman spectroscopy. For example, a duration tₑₓₚ of the measurement frame may be 100 ms (milliseconds) or more, 250 ms or more, 500 ms or more, or 750 ms or more. On the other hand, the duration tₑₓₚ of the measurement frame may be 1000 ms or less, 750 ms or less, 500 ms or less, 250 ms or less, or 100 ms or less.

The measurement frame comprises (is formed by) a plurality of measurement cycles. The measurement cycles are discrete operational phases within the measurement frame during which the apparatus 100 emits the excitation light 111 and measures induced Raman light 112. The measurement cycle is repeated multiple times within the measurement frame to enable cumulative signal acquisition for Raman spectroscopy. Any number N ≥ 2 of measurement cycles may be used. For example, the measurement frame may comprise 10 or more, 50 or more, 100 or more, 250 or more, 500 or more, or 750 or more measurement cycles. On the other hand, the measurement frame may comprise 1000 or less, 750 or less, 500 or less, 250 or less, or 100 or less measurement cycles. The duration *t_{c}* of a single measurement cycle may, hence, be *t_{c} = tₑₓₚ*/*N.*

Apart from the characteristic Raman light 112, undesired light components such as autofluorescence light 113 are caused by the excitation light 111. Autofluorescence is the natural emission of light by a material or biological tissue when it absorbs light, such as the excitation light 111, and then re-emits it at a different, e.g., longer wavelength. This inherent fluorescence, caused by the presence of endogenous fluorophores (such as certain proteins, lipids, or vitamins), appears as a broad background signal and interferes with the Raman light 112. In other words, the undesired autofluorescence overlaps with the desired Raman light. This is illustrated in **Fig. 2****.** The abscissa of diagram 200 illustrated in Fig. 2 denotes the Raman shift and the ordinate denotes the light intensity. An exemplary intensity course of the Raman signal 112 is shown in Fig. 2. The curve 210 illustrates the total intensity of the undesired autofluorescence light 113 and the Raman signal 112. As can be seen from Fig. 2, the Raman signal 112 is "buried" under the autofluorescence light 113.

As will be described in the following in greater detail, the apparatus 100 is configured to measure the incident Raman signal 112 from the sample 101 by suppressing the influence of the incident autofluorescence light 113 and other undesired incident light such as background (ambient) light 102 on the measurement. The background light 102 is the ambient illumination present in the measurement environment that is not part of the Raman signal 112. The background light 102 is characterized by a constant or slowly changing level throughout the measurement frame - meaning its intensity remains essentially stable.

Returning to Fig. 1, the apparatus 100 comprises a pixel arrangement 120. The pixel arrangement 120 may be understood as a sensor or imager configured to capture and process incident light in a way that enhances the (weak) Raman light 112 while suppressing the influence of the incident autofluorescence light 113 and the incident background light 102.

The pixel arrangement 120 comprises at least one photo-sensitive pixel. In the example of Fig. 1, two photo-sensitive pixels 121 and 122 are illustrated to highlight the structure and the operating principle of the apparatus 100. However, it should be noted that the present disclosure is not limited thereto. The pixel arrangement 120 may comprise any number M ≥ 1 of photo-sensitive pixels. The at least one photo-sensitive pixel 121, 122 may be a semiconductor device capable of converting incident light into charge carriers (e.g., electrons). For example, the at least one photo-sensitive pixel 121, 122 may be or comprise a Photonic Mixer Device (PMD), a Charge-Coupled Device (CCD), a Single-Photon Avalanche Diode (SPAD) or a Complementary Metal-Oxide-Semiconductor (CMOS) image sensor pixel. The at least one photo-sensitive pixel 121, 122 may be sensitive to the optical wavelengths of the excitation light 111, particularly in the visible and near-infrared ranges.

The pixel arrangement 120 further comprises a first charge storage 123 and a second charge storage 124. The charge storages 123 and 124 may, e.g., be capacitors or wells (e.g., deep N-wells or P-wells) within the pixel arrangement 120. The charge storages 123 and 124 may be integrated into one of the at least one photo-sensitive pixel 121, 122 in some examples. In other examples, the charge storages 123 and 124 may be elements separate from the at least one photo-sensitive pixel 121, 122.

The two charge storages 123 and 124 have respective charge levels configured to vary in response to interactions with charge carriers generated by the pixel arrangement 120. In other words, each of the two charge storages 123 and 124 has an associated charge level, represented as an electrical potential (voltage) that directly correlates to the number of accumulated charge carriers (e.g., electrons). The at least one photo-sensitive pixel 121, 122 generates charge carriers (electrons) in response to incident photons (incident light) arriving at the at least one photo-sensitive pixel 121, 122. The charge carriers are controlled by the pixel arrangement 120 to interact with the charge storages 123 and 124 in response to the excitation light 111 based on at least one control signal 103. In other words, the generated charge carriers are managed or routed by the pixel arrangement 120 based on the at least one control signal 103. The movement and collection into the first and second charge storages 123 and 124 are not arbitrary but controlled via the at least one control signal 103. The generated charge carriers are routed into either the first or second charge storage 123, 124 by the pixel arrangement 120 for integration as will be described later in greater detail. For example, the pixel arrangement 120 may comprise electronic elements such as transistors, gates or switches that actively control, based on the at least one control signal 103, whether the charge carriers are directed toward one or the other charge storage. Thus, the "interaction" may be understood as essentially the controlled routing of the charge carriers into the charge storages 123 and 124 to accumulate and measure charge. The at least one control signal 103 defines when and how the charge carriers should be routed or collected in the different charge storages 123 and 124. It effectively acts as a timing and switching reference. The charge carriers routed to one of the two charge storages 123 and 124 vary (change, modify) the charge level or voltage of the respective charge storage (e.g., the charge level or voltage may be decreased). The charge levels of both charge storages 123 and 124 are identical at the beginning of measurement frame (e.g., the charge levels are at a predetermined reset charge level).

In particular, the at least one control signal 103 is configured to control the pixel arrangement 120 in each measurement cycle to:
a) cause the charge carriers to vary the charge level of (e.g., only, exclusively) the first charge storage 123 during an initial first phase of the respective measurement cycle for measuring background light;
b) cause the charge carriers to vary the charge level of (e.g., only, exclusively) the second charge storage 124 during a second phase of the respective measurement cycle following the first phase for measuring the Raman light 112 generated in response to the excitation light 111, the excitation light 111 being emitted during the second phase of the respective measurement cycle; and
c) drain the charge carriers from the at least one photo-sensitive pixel 121, 122 during a third phase of the respective measurement cycle extending from the end of the second phase through to the end of the respective measurement cycle.

Raman scattering occurs rapidly (e.g., within about 5 to 10 picoseconds) immediately after excitation. The second phase commences (starts) before the start of the emission of excitation light 111. For example, the illumination source 110 may be configured (controlled) to emit the respective excitation light 111 at the beginning of the respective second phase (e.g., within the first 10 % or 20 % respective second phase's duration). This timing synchronization may allow to define a consistent and predictable temporal relationship between the excitation event and the subsequent detection of the Raman light 112. By ensuring excitation occurs at the start, the apparatus 100 may precisely control the timing window for Raman detection, capturing the Raman light 112 exactly when it occurs.

The at least one control signal 103 controls the pixel arrangement 120 to route the generated charge carriers (e.g., only, exclusively) to the second charge storage 124 during the second phase, ensuring that the charge carriers caused by the Raman scattering event directly vary the charge level of (e.g., only, exclusively) the second charge storage 124 (e.g., decrease voltage of the second charge storage 124 as charge is removed from it by the photocurrent caused by the charge carriers generated in response to the Raman scattering event). In other words, the at least one control signal 103 controls the pixel arrangement 120 to generate a field that exerts a force dragging (pulling) the charge carriers (e.g., only, exclusively) toward the second charge storage 124 during the second phase, ensuring that charge carriers resulting from the Raman scattering event directly vary the charge level of (e.g., only, exclusively) the second charge storage 124. Accordingly, the second charge storage 124 primarily records the Raman signal 112 plus the unavoidable background light 102. A length (duration) of the second phase may, e.g., be set based on an expected duration of the Raman light 112 generated in response to the excitation light 111 and such that the second phase ends before significant autofluorescence arrives at the pixel arrangement 120. The expected duration may, e.g., be 1 to 2 ns (nanoseconds). In other words, the length of the second phase may be 1 to 2 ns (in particular 1 ns or 2 ns). By integrating the generated charge carriers in the second phase into (e.g., only, exclusively) the second charge storage 124, the pixel arrangement 120 captures the Raman light 112 efficiently and distinguishes it temporally from the slower autofluorescence emission.

The at least one control signal 103 controls the pixel arrangement 120 to route the generated charge carriers (e.g., only, exclusively) to the first charge storage 123 during the initial first phase, ensuring that the charge carriers caused by the background light 102 directly vary the charge level of (e.g., only, exclusively) the first charge storage 123 (e.g., decrease voltage of the first charge storage 123 as charge is removed from it by the photocurrent caused by the charge carriers generated in response to the background light 102). In other words, the at least one control signal 103 controls the pixel arrangement 120 to generate a field that exerts a force dragging (pulling) the charge carriers (e.g., only, exclusively) toward the first charge storage 123 during the first phase, ensuring that charge carriers resulting from the background light 102 directly vary the charge level of (e.g., only, exclusively) the first charge storage 123. Accordingly, the first charge storage 123 primarily records the unavoidable background light 102. For example, first phase may commence (start) at the beginning of (together with) the respective measurement cycle. The first phase may end (e.g., immediately) before the start of the second phase and, hence, before the start of the emission of the excitation light 111.

The at least one control signal 103 further controls the pixel arrangement 120 to drain the charge carriers from the at least one photo-sensitive pixel 121, 122 during the third phase. The pixel arrangement 120 is configured to prevent interactions of the first and second charge storages 123 and 124 with the charge carriers drained from the at least one photo-sensitive pixel 121, 122 during the third phase. For example, the at least one control signal 103 may control the pixel arrangement 120 to route the drained charge carriers such that they do not reach the first and second charge storages 123 and 124 (e.g., route them to ground). In other words, the at least one control signal 103 controls the pixel arrangement 120 to generate a field that exerts a force dragging (pulling) the charge carriers (e.g., only, exclusively) during the third phase, ensuring that charge carriers do not reach the first and second charge storages 123 and 124. In some examples, each of the at least one photo-sensitive pixel 121, 122 may comprise a respective drain terminal (e.g., a drain gate) and the at least one control signal 103 may control the pixel arrangement 120 to drain the charge carriers from the at least one photo-sensitive pixel 121, 122 via the drain terminal of the respective photo-sensitive pixel 121, 122 such that the generated charge carriers do not reach the first and second charge storages 123 and 124. The charge carriers generated during the third phase primarily result from the slower-decaying autofluorescence light 113 and the background light 102. The emission of the Raman light 112 may have ended before the start of the third phase, such that no additional charge carriers are generated during the third phase as a result of the Raman scattering event. Because the charge carriers are drained during the third phase, the charge storages 123 and 124 do not integrate the undesired incident light (background light 102 and autofluorescence light 113). The third phase extends from the end of the second phase through to the end of the respective measurement cycle such that substantially all charge carriers resulting from the autofluorescence light 113 (and the background light 102 during the third phase) are drained from the at least one photo-sensitive pixel 121, 122 and do not interact with the first and second charge storages 123 and 124. In other words, the charge levels of the charge storages 123 and 124 are not affected by the unwanted autofluorescence light 113. A length (duration) of the third phase may, e.g., be set based on an expected duration of (the decay of) the autofluorescence light 112 generated in response to the excitation light 111. The expected duration may, e.g., be 10 to 100 ns. In other words, the length of the third phase may be 10 to 100 ns.

Controlling the pixel arrangement 120 via the at least one control signal 103 as described above enables temporal isolation of the Raman light 112 from the autofluorescence light 113. As the Raman light 112 occurs almost instantaneously after excitation, the capturing of the charge carriers in (e.g., only, exclusively) the second charge storage 124 during the second phase allows to isolate the valuable immediate signal from delayed autofluorescence. The draining of charge carriers during the third phase prevents the charge storages 123 and 124 from integrating the autofluorescence light 113. It should be noted thatintegration of the autofluorescence light 113 may be entirely omitted according to examples of the present disclosure. In other examples, a (negligible) amount of residual autofluorescence light 113 may be integrated. Furthermore, the capturing of the charge carriers in (e.g., only, exclusively) the first charge storage 123 during the first phase allows to measure the background light 102. For example, the first phase and the second phase may be of equal length (duration) such that the variation of the charge levels of the first and second charge storages 123 and 124 caused by the background light 102 is (substantially) identical. Accordingly, the background light 102 substantially cancels out when looking at the difference between the charge levels of first and second charge storages 123 and 124.

An output 125 of the pixel arrangement 120 for the measurement frame is based on a voltage difference *Vₒᵤₜ* between the first and the second charge storage 123, 124 at the end of the measurement frame: ${V}_{out} = {V}_{cs 2} - {V}_{cs 1}$ (1), with *V*_{*cs*1} denoting the voltage (charge level) of the charge storage 123 at the end of the measurement frame, and *V*_{*cs*2} denoting the voltage (charge level) of the charge storage 124 at the end of the measurement frame. The output 125 may be an analog output (e.g., an analog signal) or a digital output (e.g., digital data).

The voltages *V*_{*cs*1} and *V*_{*cs*2} represent the integrated charges, proportional to the total number of photons (signal and noise photons) received over the measurement frame. Instead of directly using the absolute signal levels (voltages or charge values) stored separately in the two charge storages 123 and 124, the difference between them after the entire measurement frame (comprising multiple measurement cycles) is determined by the pixel arrangement 120. Both charge storages 123 and 124 integrate the background light 102 similarly (common mode), meaning this unwanted signal appears almost equally in both charge storages 123 and 124. By calculating the voltage difference *Vₒᵤₜ*, this common unwanted signal effectively cancels out, isolating the Raman signal. The autofluorescence light 113 is not integrated as the charge carriers caused by the autofluorescence light 113 are drained during the third phase. Hence, the voltage difference *Vₒᵤₜ* is not affected (influenced) by the autofluorescence light 113. The differential measurement significantly enhances the Signal-to-Noise Ratio (SNR) by removing noise components common to both charge storages 123 and 124, thus amplifying the relative visibility of the Raman signal. Because the background light 102 varies slowly and appears similarly in both charge storages 123 and 124, fluctuations only minimally affect the voltage difference *Vₒᵤₜ.*

The apparatus 100 enables a time-gated signal capture and isolation of the Raman light 112. Accordingly, the apparatus 100 enables accurate Raman signal detection. Without suppression of the influence of the autofluorescence on the voltage difference *Vₒᵤₜ* between the first and the second charge storage 123, 124, the Raman signal would be lost. In other words, without draining the charge carriers caused by the autofluorescence light 113 and, hence, preventing them from interacting with the charge storages 123 and 124, both charge storages 123 and 124 would be immediately saturated by the autofluorescence and the Raman signal would be lost. The apparatus 100 may be implemented using existing sensor technologies, such as CMOS, CCD or PMD-based pixel designs. This may reduce the cost and complexity of manufacturing compared to expensive scientific cameras or advanced filters. Furthermore, the apparatus 100 may be compact compared to conventional architectures like scientific cameras making it suitable for compact, cost-efficient applications such as handheld and wearable devices (e.g., mobile phones, tablet-computers, smart watches or other wearable devices).

The pixel arrangement 120 may be configured to retain the charge levels of the charge storages 123 and 124 at the end of each measurement cycle and use them as starting charge levels for the following measurement cycle. In other words, the charges are not reset to zero or the reset charge level after each measurement cycle. At the beginning of the next measurement cycle within the same measurement frame, these previously retained charge levels serve as starting points or baseline levels. In other words, each subsequent measurement cycle builds upon the accumulated charges from prior measurement cycles, continuously integrating photon-generated charge carriers throughout multiple cycles of one measurement frame. The charge carriers accumulated over each measurement cycle within a measurement frame are cumulative. The output 125 of the pixel arrangement 120 for the measurement frame, thus, inherently integrates the photons/incident light received throughout multiple measurement cycles, effectively amplifying the weak Raman light 112 and improving signal clarity. Weak Raman signals, which may be difficult to distinguish from noise in a single short measurement cycle, may be accumulated progressively over several measurement cycles. This cumulative process substantially improves the sensitivity and detectability of very faint Raman signals. By using cumulative integration rather than resetting after each measurement cycle, the apparatus 100 may inherently average out random fluctuations (noise) over time, improving the effective SNR and thus increasing the quality and detectability of the Raman signals.

According to examples, the apparatus 100 may comprise optics 140. The optics 140 may be configured to focus the excitation light 111 onto the sample 101. The optics 140 may be arranged between the illumination source 110 and the sample 101. By focusing the excitation light 110, the photon density at the sample 101 may be increased, which boosts the probability of Raman scattering events. For example, the optics 140 may be configured to concentrate the excitation light onto a (e.g., small) defined area of the sample 101. Tightly focused excitation light may ensure that only a specific region of the sample 101 is illuminated, improving the precision of the Raman measurement.

Alternatively or additionally, the optics 140 may be configured to focus incident light (including the Raman light 112, the autofluorescence light 113 and the background light 102) onto the at least one photo-sensitive pixel 121, 122. As described above, the at least one photo-sensitive pixel 121, 122 is configured to generate the charge carriers based on the incident light. In other words, the optics 140 may be configured to collect the scattered Raman light 112 returning from the sample 101 and focus it (precisely) onto the at least one photo-sensitive pixel 121, 122. Focusing ensures improved efficiency in capturing the scattered photons that form the measurement signal. Because Raman scattering events are very rare (typically only about 1 in 10⁷ incident photons undergo Raman scattering), efficient collection and focusing is beneficial to maximize detection sensitivity.

In case the pixel arrangement 120 comprises multiple photo-sensitive pixels, the optics 140 may be configured to direct different wavelengths of the incident light onto different ones the multiple photo-sensitive pixels. In other words, the optics 140 may be configured to spatially separate the photons of the incident light according to their wavelengths (i.e., their Raman shifts). Each wavelength of the Raman light 112 may be thus directed onto a distinct area or photo-sensitive pixel of the pixel arrangement 120. Consequently, different photo-sensitive pixels of the pixel arrangement 120 detect photons of the incident light at different Raman-shifted wavelengths, allowing direct measurement of the Raman spectrum. To support this measurement approach, the pixel arrangement 120 may include multiple pairs of charge storages corresponding to the multiple photo-sensitive pixels, wherein the charge levels of each pair of storages are varied by charge carriers generated specifically by photons detected at their respective wavelengths - analogously to what is described herein for the charge storages 123 and 124. The voltage differences measured between each pair of charge storages therefore represent intensity values at specific wavelengths, collectively forming the Raman spectrum of the sample. Accordingly, the output 125 may indicate voltage differences representing intensity values of the Raman light 112 at different (specific) wavelengths.

Further alternatively or additionally, the optics 140 may be configured to block components of the incident light having the same wavelength as the excitation light 111. The illumination source 110 produces the excitation light 111 at a fixed wavelength (e.g., 532 nm, 785 nm, or 1064 nm). If not blocked, the excitation light 111 could saturate the at least one photo-sensitive pixel 121, 122, obscuring the much weaker Raman light 112. A Raman scattered photon, i.e., the Raman light 112, has a shifted wavelength (due to the energy transfer from molecular vibrations), different from the original excitation wavelength. Blocking the excitation wavelength ensures the at least one photo-sensitive pixel 121, 122 detects predominantly scattered Raman-shifted photons and helps to improve the SNR and measurement accuracy.

The optics 140 may comprise various optical elements such as at least one collimator for collimating the excitation light 111 and/or at least one focusing element (e.g., one or more lenses or lens systems) for focusing/concentrating the excitation light 111 onto the sample 101 and/or at least one focusing element (e.g., one or more lenses or lens systems) for focusing/concentrating the incident light onto the at least one photo-sensitive pixel 121, 122 and/or at least one dispersive element (e.g., a diffraction grating or a prism) configured to direct different wavelengths of the incident light onto different photo-sensitive pixels and/or at least one filter (e.g., at least one of a notch filter, an edge filter or a bandpass filter) for blocking components of the incident light having the same wavelength as the excitation light 111 and/or at least one dichroic beam splitter for blocking components of the incident light having the same wavelength as the excitation light 111.

In some examples, the apparatus 100 may be configured to conduct a second measurement in a second measurement frame. The second measurement frame is subsequent to the first measurement frame described above (i.e., it follows the first measurement frame in time). Like the first measurement frame, the second measurement frame comprises a plurality of measurement cycles. The duration (length) of the second measurement frame may be equal to the duration of the first measurement frame. Accordingly, the number and duration of measurement cycles in the second measurement frame may be equal to the number and duration of the measurement cycles in the first measurement frame. Similarly to the measurement cycles in the first measurement frame, an initial first phase, a subsequent second phase, and a concluding third phase is defined for the measurement cycles in the second measurement frame.

During the second measurement frame, the at least one control signal 103 is partly inverted compared to the first measurement frame to control the pixel arrangement 120 to cause the charge carriers to vary the charge level of (e.g., exclusively, only) the second charge storage 124 during the first phase of the respective measurement cycle. During the second phase of the respective measurement cycle in the second measurement frame, the at least one control signal 103 is configured to control the pixel arrangement 120 to cause the charge carriers to vary the charge level of (e.g., exclusively, only) the first charge storage 123. Furthermore, the at least one control signal 103 is configured to control the pixel arrangement 120 to drain the charge carriers from the at least one photo-sensitive pixel during the third phase of the respective measurement cycle in the second measurement frame. The output 125 of the pixel arrangement 120 for the second measurement frame is based on a voltage difference between the charge storages 123 and 124 at the end of the second measurement frame.

The second measurement provides an independent but complementary measurement. Combining these two measurements may reduce systematic errors and residual artifacts caused by timing imperfections. Furthermore, the reliability, accuracy, and repeatability of the Raman spectroscopy measurements may be improved.

According to examples, the apparatus 100 may further comprise processing circuitry 130. For example, the processing circuitry 130 may be a single dedicated processor, a single shared processor, or a plurality of individual processors, some of which or all of which may be shared, a digital signal processor (DSP) hardware, an application specific integrated circuit (ASIC), a system-on-a-chip (SoC), a neuromorphic processor or a field programmable gate array (FPGA). The processing circuitry 130 may optionally be coupled to, e.g., memory such as read only memory (ROM) for storing software, random access memory (RAM) and/or non-volatile memory. For example, the apparatus 100 may comprise memory configured to store instructions, which when executed by the processing circuitry 130, cause the processing circuitry 130 to perform the steps and methods described herein.

In particular, the processing circuitry 130 may be configured to determine a characteristic of the sample 101 based on the output 125 of the pixel arrangement 120 for at least the (first) measurement frame. In other words, the processing circuitry 130 may be configured to convert the differential output 125 (which primarily represents the Raman-scattered signal due to the suppression of the influence of the autofluorescence and ambient signals) into meaningful analytical results, i.e., the determination of the characteristic of the analyzed sample 101. The characteristic of the sample 101 refers to a physical, chemical, or biological property of the sample 101. The voltage difference represented by the output 125 reflects primarily the intensity of Raman-scattered photons at specific wavelengths, having suppressed the influence of unwanted signals, such as background (ambient) light or autofluorescence. As described above, the output 125 may indicate voltage differences representing intensity values of the Raman light 112 at one or more different (specific) wavelengths. Accordingly, the processing circuitry 130 may be configured to determine a spectrum of the Raman light 112 based on the output 125 of the pixel arrangement 120 for at least the (first) measurement frame. The spectrum of the Raman light 112 is a spectral fingerprint unique to the sample 101. By comparing the spectrum of the Raman light 112 against known spectral signatures of known molecules, etc., the processing circuitry 130 may determine the characteristic of the sample. The processing by the processing circuitry 130 may comprise various other aspects such as analog-to-digital conversion, filtering, baseline correction, normalization, smoothing, peak detection, etc.

The processing circuitry 130 enables real-time analysis and determination of relevant characteristics of the sample 101 directly within the apparatus 100, eliminating the need for external analysis equipment or post-processing steps.

In case a second measurement is performed, the processing circuitry 130 may be configured to determine the characteristic of the sample 101 based on an average determined based on (e.g., an average of) of the outputs 125 of the pixel arrangement 125 for the first measurement frame and the second measurement frame. The averaging allows to statistically cancel out systematic measurement errors and common-mode background signals that are still present after each individual measurement. This improves the isolation of the Raman light 112 from the background light 102. Using averaged outputs ensures robustness against transient background fluctuations, systematic measurement errors or other measurement instabilities. Averaging multiple complementary frames reduces random fluctuations and noise, significantly enhancing the clarity and visibility of the weak Raman light 112.

According to examples, the sample 101 may be an organism (such as a human or human tissue) and the characteristic of the sample 101 may be a physiological property of the organism. Raman spectroscopy provides distinctive molecular fingerprints, enabling precise detection of biochemical substances, even in complex biological environments. A use case for the apparatus 100 may be non-invasive in-vivo blood glucose or lactate measurement. The apparatus 100's unique ability to effectively suppress the influence of the strong autofluorescence typically found in biological tissues (such as human skin and blood) and accurately detect the weak Raman signals allows reliable determination of physiological parameters, such as glucose or lactate concentration. In other words, the physiological property may be at least one of a blood glucose value or a lactate value of the organism. This capability is highly beneficial for medical applications, such as wearable devices used by diabetes patients or athletes, enhancing the usability and accuracy of health monitoring devices in real-world conditions.

In other examples, the sample 101 may be foodstuff and the characteristic of the sample 101 may be a foodstuff property. Raman spectroscopy allows to detect and quantify specific molecular components in food products, such as sugars (e.g., fructose and glucose), protein content, lipid content and contaminants, by identifying their characteristic Raman spectrum. By effectively suppressing the influence of autofluorescence that is commonly present in biological materials like fruits, vegetables, juices, and other organic food products, the apparatus 100 enables accurate determination of food-related properties, such as ripeness, freshness, sugar content, or potential defects. This capability is highly beneficial in the food industry, agriculture, and consumer markets, allowing rapid, non-destructive, and precise quality assessments directly in portable or handheld formats. Thus, the proposed technology allows to significantly improve the reliability and efficiency of food-quality control, enabling advanced, cost-effective analytical solutions previously difficult to realize due to autofluorescence interference.

It should be noted that the processing circuitry 130 is an optional element of the apparatus 100. In alternative examples, the output 125 of the pixel arrangement 120 for at least the (first) measurement frame may be processed by circuitry external to the apparatus 100 (e.g., according to the principles described above). In these examples, the apparatus 100 may instead of the processing circuitry 130 comprise an interface configured to output the output 125 of the pixel arrangement 120 for at least the (first) measurement frame to external circuitry.

The at least one control signal 103 and the drive signal 104 may, e.g., be generated (provided) by the processing circuitry 130 or a separate control circuitry (not illustrated in Fig. 1) of the apparatus 100. For example, the at least one control signal 103 and the drive signal 104 may be generated based on control data (control instructions) stored in a memory of the apparatus 100.

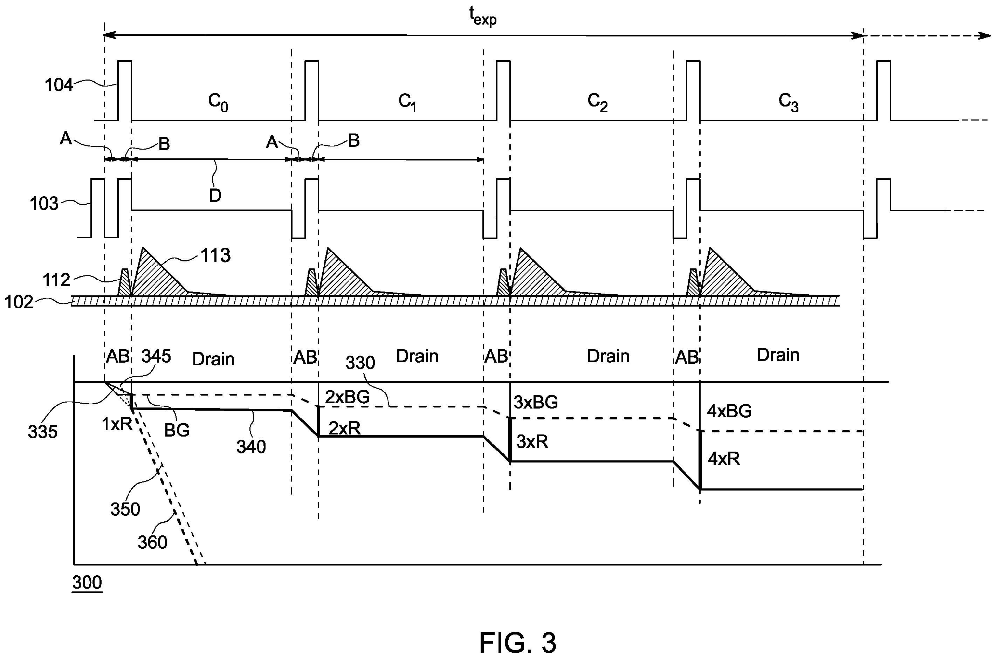

As described above, the at least one photo-sensitive pixel 121, 122 may be implemented in various ways. In the following, a first exemplary implementation will be described with reference to Fig. 3. In the first exemplary implementation, the at least one photo-sensitive pixel 121, 122 is configured to separate the charge carriers generated in the photo-sensitive pixel 121, 122 the measurement frame based on the at least one control signal 103. The at least one control signal 103 is used to control the separation of the charge carriers generated in the photo-sensitive pixel 121, 122 as well as the draining during the measurement frame(s).

For example, the at least one photo-sensitive pixel 121, 122 may comprise a PMD, a CCD or a SPAD to separate, based on the at least one control signal 103, the charge carriers generated in the photo-sensitive pixel 121, 122 during the measurement frame(s). The at least one photo-sensitive pixel 121, 122 may comprise internal control structures - such as gated transistors, switches or electric potentials - controlled by the at least one control signal 103 to selectively guide and direct the generated charge carriers into one of the two available storages 123 and 124 or to drain the generated charge carriers based on timing information provided by the at least one control signal 103. The internal control structures dynamically alter the pixel's internal electric potential based on the at least one control signal 103 to selectively separate the generated charge carriers and route them into one of the two available storages 123 and 124 or to drain the generated charge carriers from the photo-sensitive pixel. Consequently, the pixel arrangement 120 may integrate charge carriers separately into each charge storage 123, 124 at predefined phases within each measurement cycle as well as drain charge carriers at another predefined phase within each measurement cycle. This dynamic routing enables the at least one photo-sensitive pixel 121, 122 to perform time-gated integration and draining, selectively capturing the desired photons of the Raman light 112 while effectively suppressing the influence of the unwanted photons from the autofluorescence light 113 and the background light 102. For example, the pixel arrangement 120 may be or comprise a Time-of-Flight (ToF) sensor such that the at least one photo-sensitive pixel 121, 122 is at least one photo-sensitive pixel of the ToF sensor. The at least one photo-sensitive pixel 121, 122 may, e.g., comprise control gates for controlling the flow of the generated charge carriers inside the photo-sensitive sensor pixel (e.g., two or more modulation gates and one or more drain gates) and read-out terminals for coupling the at least one photo-sensitive pixel 121, 122 with the two charge storages 123 and 124.

This will be explained in greater detail in the following with reference to timing diagram 300 illustrated in **Fig. 3****.** In the example of Fig. 3, four measurement cycles C₀, C₁, C₂ and C₃ are exemplarily illustrated. However, as described above, any number N ≥ 2 of measurement cycles may be used.

The emission times of the excitation light 111 by the illumination source 110 are illustrated in Fig. 3 via the drive signal 104. As described above, the drive signal 104 drives the illumination source 110 and controls the emission of the excitation light 111 by the illumination source 110. When the (signal level of the) drive signal 104 is high, the illumination source 110 emits the excitation light 111. As can be seen from the signal shape of the drive signal 104, respective excitation light 111 is emitted in each of the depicted measurement cycles C₀ to C₃.

An exemplary temporal course (shape) of the control signal 103 is illustrated in Fig. 3. When the (signal level of the) control signal 103 is low, the at least one photo-sensitive pixel 121, 122 separates the charge carriers to vary the charge level of (e.g., only, exclusively) the first charge storage 123. When the (signal level of the) control signal 103 is high, the at least one photo-sensitive pixel 121, 122 separates the charge carriers to vary the charge level of (e.g., only, exclusively) the second charge storage 124. When the signal level of the control signal 103 is middle (medium. i.e., at a defined level between high and low), the at least one photo-sensitive pixel 121, 122 drains the charge carriers such that the charge carriers are prevented from interacting with the charge storages 123 and 124. However, it should be noted that in alternative examples the response of the at least one photo-sensitive pixel 121, 122 to the signal levels of the control signal 103 may be different (e.g., reversed). It should be further noted that instead of using a single control signal with three signal levels, two separate control signals may be used. A first control signal may be used to control the separation of the charge carriers generated in the photo-sensitive pixel 121, 122 as well as their routing to the charge storages 123 and 124. For example, when the first control signal is low, the at least one photo-sensitive pixel 121, 122 may separate the charge carriers to vary the charge level of (e.g., only, exclusively) the first charge storage 123. When the first control signal is high, the at least one photo-sensitive pixel 121, 122 may separate the charge carriers to vary the charge level of (e.g., only, exclusively) the second charge storage 124. A second control signal may be used to control the charge carrier drainage. For example, when the second control signal is low, the at least one photo-sensitive pixel 121, 122 may separate the charge carriers to vary the charge level of one of the charge storages 123 and 124 based on the first control signal. When the second control signal is high, the at least one photo-sensitive pixel 121, 122 may drain the charge carriers. For ease of explanation, control of the at least one photo-sensitive pixel 121, 122 via the single control signal 103 will be described in the following.

The above described control of the illumination source 110 and the at least one photo-sensitive pixel 121, 122 will be described exemplarily for the first measurement cycle C₀ illustrated in Fig. 3.

During the first phase A of the first measurement cycle C₀ which initiates (begins) concurrently with the measurement frame in the example of Fig. 3, the control signal 103 is low to control the at least one photo-sensitive pixel 121, 122 to separate the charge carriers to vary the charge level of (e.g., only, exclusively) the first charge storage 123. No excitation light is emitted during the first phase A as indicated by the drive signal 104. Only the background light 102 is incident on the at least one photo-sensitive pixel 121, 122. Accordingly, the background light 102 is measured by the at least one photo-sensitive pixel 121, 122 and integrated into the first charge storage 123.

At the beginning of the second phase B of the first measurement cycle C₀, the illumination source 110 is configured (controlled) to emit the excitation light 111. The emitted excitation light 111 interacts immediately (essentially concurrently with the start of the emission of excitation light 111) with the molecular structure of the sample 101, resulting in the inelastic scattering of photons. The Raman light 112 emitted due to the Raman scattering is incident on the at least one photo-sensitive pixel 121, 122.

During the second phase B of the first measurement cycle C₀ which initiates (begins) essentially concurrently with the emission of the excitation light 111 in the example of Fig. 3, the control signal 103 is high to control the at least one photo-sensitive pixel 121, 122 to separate the charge carriers to vary the charge level of (e.g., only, exclusively) the second charge storage 124. Accordingly, the Raman light 112 generated in response to the excitation light 111 is measured by the at least one photo-sensitive pixel 121, 122 and integrated into the second charge storage 124. The length of the second phase B may, e.g., be set based on an expected duration of the Raman light 112 generated in response to the excitation light 111 and such that the second phase B ends before significant autofluorescence arrives at the at least one photo-sensitive pixel 121, 122. For example, the length of the second phase B may be set to 1 or 2 ns.

The autofluorescence light 113 caused by the excitation light 111 is incident on the at least one photo-sensitive pixel 121, 122 immediately after the Raman light 112. During the third phase D of the first measurement cycle C₀ which immediately follows the second phase B, the signal level the control signal 103 is middle to control the pixel arrangement 120 to drain the charge carriers from the at least one photo-sensitive pixel 121, 122. The pixel arrangement 120 prevents interactions of the first and second charge storages 123 and 124 with the charge carriers drained from the at least one photo-sensitive pixel 121, 122 during the third phase. The third phase extends from the end of the second phase through to the end of the first measurement cycle C₀ such that substantially all charge carriers resulting from the autofluorescence light 113 (plus the charge carriers resulting from the background light 102 incident during the third phase D) are drained from the at least one photo-sensitive pixel 121, 122 and do not interact with the first and second charge storages 123 and 124. Accordingly, the charge levels of the charge storages 123 and 124 are not affected by the unwanted autofluorescence light 113. The length of the third phase may, e.g., be set based on an expected duration of (the decay of) the autofluorescence light 112 generated in response to the excitation light 111. For example, the length of the third phase D may be set to 10 to 100 ns.

The charge levels of the first charge storage 123 and the second charge storage 124 are exemplarily illustrated in the lower part of Fig. 3. Curve 330 shows the temporal course of the charge level of the first charge storage 123 over the measurement frame. Curve 335 shows the temporal course of the charge level of the first charge storage 123 in greater detail for the first measurement cycle C₀. Curve 340 shows the temporal course of the charge level of the second charge storage 124 over the measurement frame. Curve 345 shows the temporal course of charge level of the second charge storage 124 in greater detail for the first measurement cycle C₀. The charge levels of both charge storages 123 and 124 are identical at the beginning of the measurement frame (e.g., the charge levels are at a predetermined reset charge level).

During the first phase A of the first measurement cycle C₀, only the background light 102 is incident on the at least one photo-sensitive pixel 121, 122. As the control signal 103 is low during the first phase A of the first measurement cycle C₀, the at least one photo-sensitive pixel 121, 122 is controlled to separate the charge carriers to vary (e.g., lower) the charge level of (e.g., only, exclusively) the first charge storage 123. During the second phase B of the first measurement cycle C₀. As the control signal 103 is high during the second phase B of the first measurement cycle C₀, the at least one photo-sensitive pixel 121, 122 is controlled to separate the charge carriers to vary (e.g., lower) the charge level of (e.g., only, exclusively) the second charge storage 124. In the example of Fig. 3, the charge storages 123 and 124 are "integrating down". From the beginning of the first measurement cycle C₀ up to the end of the second phase B, both charge storages 123 and 124 are integrating the background light 102 (indicated by "BG" in Fig. 3). Only the second charge storage 124 integrates the Raman light 112. Accordingly, the difference between the charge levels of the charge storages 123 and 124 at the end of the second phase B is (represents) the Raman light 112 (indicated by "R" in Fig. 3).

During the third phase D of the first measurement cycle C₀, the autofluorescence light 113 and the background light 102 are incident on the at least one photo-sensitive pixel 121, 122. As the signal level of the control signal 103 is middle, the pixel arrangement 120 is controlled to drain the charge carriers from the at least one photo-sensitive pixel 121, 122 such that the charge carriers drained from the at least one photo-sensitive pixel 121, 122 are prevented from interacting with the first and second charge storages 123 and 124. During the third phase D of the first measurement cycle C₀, neither the first charge storage 123 nor the second charge storage 124 integrates the autofluorescence light 113 and the background light 102. Accordingly, the influence of the autofluorescence light 113 is effectively suppressed. The charge levels of the charge storages 123 and 124 are not varied during the third phase D of the first measurement cycle C₀ such that the difference between the charge levels of the charge storages 123 and 124 at the end of the third phase D and, hence, at the end of the first measurement cycle C₀ is (represents) still only the Raman light 112. In other words, the difference between the charge levels of the charge storages 123 and 124 at the end of the first measurement cycle C₀ is 1 · *R* with *R* denoting the charge level difference due to (caused by) the Raman light 112.

As has become evident from the above, the apparatus 100 enables a time-gated signal capture and isolation of the Raman light 112. The draining of charge carriers during the third phase prevents the charge storages 123 and 124 from integrating the autofluorescence light 113. Accordingly, the apparatus 100 enables accurate Raman signal detection. Without draining the charge carriers caused by the autofluorescence light 113, both charge storages 123 and 124 would be immediately saturated by the autofluorescence and the Raman signal would be lost. This is exemplarily illustrated by curves 350 and 360 in Fig. 3 which show the hypothetic courses of the charge levels of the charge storages 123 and 124 without draining the charge carriers caused by the autofluorescence light 113. As the control signal 103 governs the charge separation and drainage, precise control of which charge storage receives the charges during different phases of the measurement cycle is enabled. This flexibility ensures the apparatus 100 may adapt to various signal and background conditions, optimizing performance for different types of samples. In particular, other modulation codes than the 10D... code used in the example of Fig. 3 for the control signal 103 may be used. The dual charge storage architecture with drainage functionality and reference signal control may be implemented using existing sensor technologies, such as CCD or PMD-based designs. This may reduce the cost and complexity of manufacturing compared to expensive scientific cameras or advanced filters. Furthermore, the apparatus 100 may be compact compared to conventional architectures like scientific cameras making it suitable for compact, cost-efficient applications such as handheld and wearable devices (e.g., mobile phones, tablet-computers, smart watches or other wearable devices).

In examples in which the at least one photo-sensitive pixel 121, 122 is embodied as a pixel of a ToF sensor that provides two charge storages per pixel (embodying the charge storages 123 and 124), only one of the two charge storages is, by design, electrically connected to the photosensitive junction of the pixel at any given instant. At the start of a measurement frame both the first and the second charge storages 123, 124 are reset to the same reference potential; immediately thereafter the at least one control signal 103 selects the first charge storage 123 for integration during the initial first phase A, while the second charge storage 124 is electrically isolated and therefore simply retains its reset level. Consequently, throughout phase A the first charge storage 123 integrates the constant background light 102, whereas the second charge storage 124 does not integrate any photocurrent. As described above, the first phase A terminates before the emission of the excitation light 111. The charge-level difference between the two charge storages at the end of phase A therefore represents essentially the background contribution. During the subsequent second phase B control signal 103 selects the second charge storage 124 for integration, while the first charge storage 123 is electrically isolated and therefore simply retains the charge level at the end of phase A. Consequently, throughout phase B the second charge storage 124 integrates the Raman photons together with the constant background light 102, whereas the second charge storage 124 does not integrate any photocurrent. As described above, the second phase B terminates before a measurable amount of autofluorescence reaches the ToF sensor. The charge-level difference between the two charge storages at the end of phase B therefore represents essentially only the Raman contribution. The background contribution is identical in both storages. The rapid toggling between the first and the second charge storages 123, 124 from the beginning of the measurement cycle until the end of the second phase B is an inherent timing behavior of the ToF architecture used to isolate the Raman contribution. This enforced alternation renders the background contribution substantially a common-mode component that de facto completely cancels in the differential read-out as described above and below. A drain terminal such as an integrated drain gate of the ToF sensor's pixel allows to drain the autofluorescence based on the at least one control signal 103 during the third phase D and, hence, prevents integration of the autofluorescence into the charge storages 123, 124.

In the example of Fig. 3, the at least one photo-sensitive pixel 121, 122 is configured to retain the charge levels of the two charge storages 123 and 124 at the end of each measurement cycle and use them as starting charge levels for the following measurement cycle. That is, the charge levels of the two charge storages 123 and 124 at the end of first measurement cycle C₀ are used as the starting charge levels for the second measurement cycle C₁ and so on. In other words, the charge levels of the two charge storages 123 and 124 are not reset at the end of a measurement cycle. This cumulative integration allows the apparatus 100 to build up weak Raman signals over multiple measurement cycles, significantly increasing the sensitivity of the apparatus 100 to detect low-intensity signals that would otherwise be undetectable in a single cycle. The consistent accumulation of charges may enhance the SNR, making it easier to distinguish Raman signals from background noise.

The control of the illumination source 110 and the at least one photo-sensitive pixel 121, 122 in the second measurement cycle C₁ is as described above for the first measurement cycle C₀.

During the first phase A of the second measurement cycle C₁, only the background light 102 is incident on the at least one photo-sensitive pixel 121, 122. As the at least one control signal 103 is low during the first phase A of the second measurement cycle C₁, the at least one photo-sensitive pixel 121, 122 is controlled to separate the charge carriers to vary (e.g., lower) the charge level of (e.g., only, exclusively) the first charge storage 123. As the at least one control signal 103 is high during the second phase B of the second measurement cycle C₁, the at least one photo-sensitive pixel 121, 122 is controlled to separate the charge carriers to vary (e.g., lower) the charge level of (e.g., only, exclusively) the second charge storage 124. From the beginning of the second measurement cycle C₁ up to the end of the second phase B, both charge storages 123 and 124 are again integrating the background light 102. Only the second charge storage 124 integrates the Raman light 112 - like in the first measurement cycle C₀. Accordingly, the difference between the charge levels of the charge storages 123 and 124 at the end of the second phase B of the second measurement cycle C₁ is 2 ·*R.*

In the next measurement cycles C₂, C₃, ..., the behavior is analogously. Accordingly, after *n* measurement cycles, the difference between the charge levels of the charge storages 123 and 124 is *n* · *R*. The output 125 of the pixel arrangement 120 for the measurement frame is based on the voltage difference corresponding to the charge level difference *n* · *R* between the charge storages 123 and 124.

In the examples of Fig. 3, a single photo-sensitive pixel is used to control the charge levels of the charge storages 123 and 124. However, in alternative examples, two photo-sensitive pixel may be used to control the charge levels in the charge storages 123 and 124. This allows to use simpler pixels such as conventional CMOS image sensor pixels with drain functionality. This will be described in greater detail below with reference to Fig. 1.

In these examples, the first photo-sensitive pixel 121 is configured to integrate the incident light for varying the charge level of the first charge storage 123. The second photo-sensitive pixel 122 configured to integrate the incident light for varying the charge level of the second charge storage 124. In other words, two separate photo-sensitive pixels 121 and 122 are used - one dedicated to integrating light into the first charge storage 123 and the other to the second charge storage 124. The pixel arrangement 120 is operable to drain the charge carriers from the two photo-sensitive pixels 121 and 122. The pixel arrangement 120 is operable to prevent interactions of the first and second charge storages 123 and 124 with the charge carriers drained from the two photo-sensitive pixels 121 and 122. For example, the pixel arrangement 120 may be operable to route the drained charge carriers such that they do not reach the first and second charge storages 123 and 124 (e.g., route them to ground). In other words, the pixel arrangement 120 may generate a field that exerts a force dragging (pulling) the charge carriers (e.g., only, exclusively) during the third phase, ensuring that charge carriers do not reach the first and second charge storages 123 and 124. In some examples, each of the two photo-sensitive pixels 121 and 122 may comprise a respective drain terminal (e.g., a drain gate) and the pixel arrangement 120 may be operable to drain the charge carriers from the at least one photo-sensitive pixel 121, 122 via the drain terminal of the respective photo-sensitive pixel 121, 122 such that the generated charge carriers do not reach the first and second charge storages 123 and 124.

The at least one control signal 103 is used to operate these two photo-sensitive pixels 121 and 122 in different phases of each measurement cycle. In particular, the at least one control signal 103 may be configured to:
a) control the first photo-sensitive pixel 121 to integrate the incident light during the first phase of the respective measurement cycle such that the charge carriers vary the charge level of (e.g., only, exclusively) the first charge storage 123 during the first phase;
b) control the second photo-sensitive pixel 122 to integrate the incident light during the second phase of the respective measurement cycle such that the charge carriers vary the charge level of (e.g., only, exclusively) the second charge storage 124 during the second phase; and
c) control the first and the second photo-sensitive pixel 121 and 122 to drain the charge carriers during the third phase of the respective measurement cycle such that the charge carriers do not interact with the first and the second charge storage 123 and 124.

During the first phase, the at least one control signal 103 directs the first photo-sensitive pixel 121 to integrate the incident light, which causes the charge level in (e.g., only, exclusively) the first charge storage 123 to change in response to the collected photons (for example, by discharging it). This ensures that the charge carriers caused by the background light directly vary the charge level of (e.g., only, exclusively) the first charge storage 123 (e.g., decrease voltage of the first charge storage 123 as charge is removed from it by the photocurrent caused by the charge carriers generated in response to the background light). During the second phase, the at least one control signal 103 directs the second photo-sensitive pixel 122 to integrate the incident light, which causes the charge level in (e.g., only, exclusively) the second charge storage 124 to change in response to the collected photons (for example, by discharging it). This ensures that the charge carriers caused by the Raman scattering event plus the unavoidable background light directly vary the charge level of (e.g., only, exclusively) the second charge storage 124 (e.g., decrease voltage of the second charge storage 124 as charge is removed from it by the photocurrent caused by the charge carriers generated in response to the Raman scattering event). Because both photo-sensitive pixels 121 and 122 subsequently integrate the background light 102 during the first and the second phase, the background light 102 is a common mode component for both charge storages 123 and 124 and effectively cancels out such that the difference between the charge levels of the first and second charge storages 123 and 124 at the end of the second phase represents effectively only the Raman signal.

The at least one control signal 103 further controls the pixel arrangement 120 to drain the charge carriers from the first and the second photo-sensitive pixel 121 and 122. This ensures that the charge storages 123 and 124 do not integrate the autofluorescence light 113. Accordingly, the difference between the charge levels of the first and second charge storages 123 and 124 at the end of the respective measurement cycle represents effectively only the Raman signal.

Controlling the photo-sensitive pixels 121 and 122 via the at least one control signal 103 as described above enables temporal isolation of the Raman light 112 from the autofluorescence light 112 and the background light 102. The variation of the charge levels of both charge storages 123 and 124 is analogous to what is described above with reference to Fig. 3 assuming that (e.g., only, exclusively) the first photo-sensitive pixel 121 integrates the incident light when the control signal 103 is low, and that (e.g., only, exclusively) the second photo-sensitive pixel 122 integrates the incident light when the control signal 103 is high. Analogously to what is described above, the charge carriers are drained from the first and the second photo-sensitive pixel 121 and 122 when the signal level of the control signal is middle. Analogously to what is described above, a single control signal or two control signals may be used.

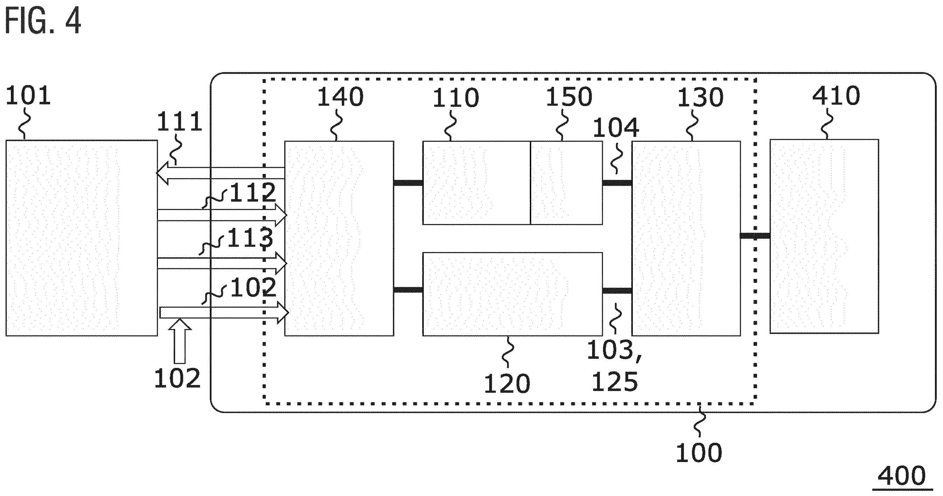

**Fig. 4** schematically illustrates a handheld or wearable device 400. For example, the handheld and wearable device 400 may be one of a mobile phone, a tablet-computer, handheld food-quality analyzer, a smart watch, a wearable medical device for non-invasive glucose monitoring, a wearable lactate measurement device (e.g., for athletes) or another wearable device. The handheld or wearable device 400 comprises an apparatus 100 for Raman spectroscopy as described herein.

The pixel arrangement 120 with dual charge storages, drain function, precise time-gated measurement, and effective suppression of the influence of the ambient and autofluorescence light 102 and 113 makes the apparatus 100 particularly suited for integration into compact, portable devices. The handheld or wearable device 400 benefits from the apparatus 100's enhanced sensitivity and accuracy, allowing robust and reliable real-time measurements of physiological parameters, such as blood glucose or lactate concentrations, or the rapid and non-invasive analysis of foodstuff properties. The practical advantages provided by the apparatus 100 - like improved accuracy, real-time feedback, low complexity, and reduced cost - allow immediate, non-destructive, and precise molecular-level assessments.

Fig. 4 further highlights that the apparatus 100 may optional further comprise driver circuitry 150 for driving the emission of the excitation light 111 by the light source 110. For example, the driver circuitry 150 may drive the light source 110 based on the drive signal 104. The drive signal 104 may be provided by the processing circuitry 130 as illustrated in Fig. 4 or alternatively by a dedicated control circuitry of the apparatus 100 (not illustrated).

According to examples, the handheld or wearable device 400 may optionally further comprise a Human-Machine Interface (HMI) 410 such as a display, in particular a touch-sensitive display. The HMI 410 allows to display or otherwise output the determined characteristic(s) of the sample 110. In other examples, the HMI 410 may allow a user to input control commands for controlling performance of the Raman spectroscopy by the apparatus 100.

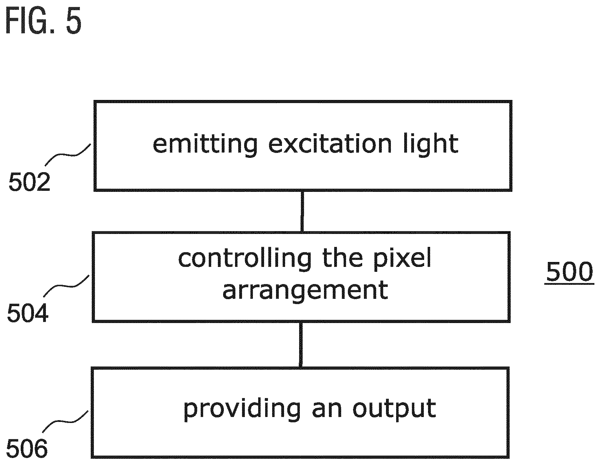

For further highlighting the Raman spectroscopy technology described above, **Fig. 5** illustrates a flowchart of a method 500 for Raman spectroscopy using a pixel arrangement. The pixel arrangement comprises at least one photo-sensitive pixel. The pixel arrangement further comprises a first charge storage and a second charge storage having respective charge levels configured to vary in response to interactions with charge carriers generated by the pixel arrangement. The method 500 comprises emitting 502, for a plurality of measurement cycles in a measurement frame, respective excitation light to induce Raman scattering in a sample. The charge carriers are controlled by the pixel arrangement to interact with the charge storages in response to the excitation light based on at least one control signal. The method 500 further comprises controlling 504 the pixel arrangement via the at least one control signal in each measurement cycle to a) cause the charge carriers to vary the charge level of the first charge storage during an initial first phase of the respective measurement cycle for measuring background light; b) cause the charge carriers to vary the charge level of the second charge storage during a second phase of the respective measurement cycle following the first phase for measuring Raman light generated in response to the excitation light, the excitation light being emitted during the second phase of the respective measurement cycle; and c) drain the charge carriers from the at least one photo-sensitive pixel during a third phase of the respective measurement cycle extending from the end of the second phase through to the end of the respective measurement cycle. The pixel arrangement prevents interactions of the first and second charge storages with the charge carriers drained from the at least one photo-sensitive pixel during the third phase. The method 500 comprises providing 506 an output of the pixel arrangement for the measurement frame based on a voltage difference between the first and the second charge storage at the end of the measurement frame.

Analogously to what is described above, the method 500 enables accurate Raman signal detection due to the time-gated signal capture and isolation of the Raman light. The method 500 may be implemented using existing sensor technologies, such as CMOS, CCD or PMD-based pixel designs or existing ToF sensors, reducing the cost and complexity compared to expensive scientific cameras.

More details and aspects of the method 500 are explained in connection with the proposed technique or one or more examples described above (e.g., Fig. 1 to Fig. 4). The method 500 may comprise one or more additional optional features corresponding to one or more aspects of the proposed technique or one or more examples described above.

As described above, the apparatus 100, the handheld or wearable device 400 as well as the method 500 may be used for various applications. Particularly, the apparatus 100, the handheld or wearable device 400, and the method 500 may be used for determining a physiological property of an organism or foodstuff analysis.

Examples of the present disclosure may provide a time-gated Raman sensor using a conventional imaging sensor such as a conventional ToF sensor with drain terminals (e.g., gates).

The examples as described herein may be summarized as follows:
An example (e.g., example 1) relates to an apparatus for Raman spectroscopy. The apparatus comprises an illumination source configured to emit, for a plurality of measurement cycles in a measurement frame, respective excitation light to induce Raman scattering in a sample. In addition, the apparatus comprises a pixel arrangement. The pixel arrangement comprises at least one photo-sensitive pixel. The pixel arrangement further comprises a first charge storage and a second charge storage having respective charge levels configured to vary in response to interactions with charge carriers generated by the pixel arrangement. The charge carriers are controlled by the pixel arrangement to interact with the charge storages in response to the excitation light based on at least one control signal. The at least one control signal is configured to control the pixel arrangement in each measurement cycle to a) cause the charge carriers to vary the charge level of the first charge storage during an initial first phase of the respective measurement cycle for measuring background light; b) cause the charge carriers to vary the charge level of the second charge storage during a second phase of the respective measurement cycle following the first phase for measuring Raman light generated in response to the excitation light, the excitation light being emitted during the second phase of the respective measurement cycle; and c) drain the charge carriers from the at least one photo-sensitive pixel during a third phase of the respective measurement cycle extending from the end of the second phase through to the end of the respective measurement cycle. The pixel arrangement is configured to prevent interactions of the first and second charge storages with the charge carriers drained from the at least one photo-sensitive pixel during the third phase. An output of the pixel arrangement for the measurement frame is based on a voltage difference between the first and the second charge storage at the end of the measurement frame.

Another example (e.g., example 2) relates to a previous example (e.g., example 1) or to any other example, wherein the pixel arrangement is configured to retain the charge levels of the charge storages at the end of each measurement cycle and use them as starting charge levels for a following measurement cycle.

Another example (e.g., example 3) relates to a previous example (e.g., one of the examples 1 or 2) or to any other example, wherein the at least one photo-sensitive pixel is configured to separate the charge carriers generated in the photo-sensitive pixel during the measurement frame based on the at least one control signal.

Another example (e.g., example 4) relates to a previous example (e.g., one of the examples 1 or 2) or to any other example, wherein the pixel arrangement comprises a first photo-sensitive pixel configured to integrate incident light for varying the charge level of the first charge storage, and a second photo-sensitive pixel configured to integrate the incident light for varying the charge level of the second charge storage, and wherein the at least one control signal is configured to control the first photo-sensitive pixel to integrate the incident light during the first phase of the respective measurement cycle such that the charge carriers vary the charge level of the first charge storage during the first phase, control the second photo-sensitive pixel to integrate the incident light during the second phase of the respective measurement cycle such that the charge carriers vary the charge level of the second charge storage during the second phase, and control the first and the second photo-sensitive pixel to drain the charge carriers during the third phase of the respective measurement cycle.

Another example (e.g., example 5) relates to a previous example (e.g., one of the examples 1 to 4) or to any other example, wherein during a second measurement frame, the at least one control signal is configured to control the pixel arrangement to cause the charge carriers to vary the charge level of the second charge storage during the first phase of the respective measurement cycle, cause the charge carriers to vary the charge level of the first charge storage during the second phase of the respective measurement cycle, and drain the charge carriers from the at least one photo-sensitive pixel during the third phase of the respective measurement cycle, and wherein an output of the pixel arrangement for the second measurement frame is based on a voltage difference between the charge storages at the end of the second measurement frame.

Another example (e.g., example 6) relates to a previous example (e.g., one of the examples 1 to 5) or to any other example, wherein the first phase and the second phase are of equal length.

Another example (e.g., example 7) relates to a previous example (e.g., one of the examples 1 to 6) or to any other example, wherein the illumination source is configured to emit the respective excitation light at the beginning of the respective second phase.

Another example (e.g., example 8) relates to a previous example (e.g., one of the examples 1 to 7) or to any other example, further comprising processing circuitry configured to determine a characteristic of the sample based on the output of the pixel arrangement for the measurement frame.

Another example (e.g., example 9) relates to a previous example (e.g., example 8 when dependent on example 5) or to any other example, wherein the processing circuitry is configured to determine a characteristic of the sample based on an average determined based on the outputs of the pixel arrangement for the measurement frame and the second measurement frame.

Another example (e.g., example 10) relates to a previous example (e.g., one of the examples 8 or 9) or to any other example, wherein the sample is an organism and the characteristic of the sample is a physiological property of the organism.

Another example (e.g., example 11) relates to a previous example (e.g., example 10) or to any other example, wherein the physiological property is at least one of a blood glucose value or a lactate value of the organism.

Another example (e.g., example 12) relates to a previous example (e.g., one of the examples 8 or 9) or to any other example, wherein the sample is foodstuff and the characteristic of the sample is a foodstuff property.

Another example (e.g., example 13) relates to a previous example (e.g., one of the examples 1 to 12) or to any other example, further comprising optics configured to focus incident light onto the at least one photo-sensitive pixel, wherein the at least one photo-sensitive pixel is configured to generate the charge carriers based on the incident light, and block components of the incident light having the same wavelength as the excitation light.

Another example (e.g., example 14) relates to a previous example (e.g., example 13) or to any other example, wherein the optics is further configured to focus the excitation light onto the sample.

Another example (e.g., example 15) relates to a previous example (e.g., one of the examples 1 to 14) or to any other example, wherein the excitation light exhibits a wavelength in the visible or near-infrared range.

Another example (e.g., example 16) relates to a previous example (e.g., one of the examples 1 to 15) or to any other example, wherein a length of the second phase is set based on expected duration of the Raman light generated in response to the excitation light.

Another example (e.g., example 17) relates to a previous example (e.g., one of the examples 1 to 16) or to any other example, wherein a length of the third phase is set based on an expected duration of autofluorescence generated by the sample in response to the excitation light.

Another example (e.g., example 18) relates to a handheld or wearable device comprising an apparatus for Raman spectroscopy according to a previous example (e.g., one of the examples 1 to 17) or to any other example.

An example (e.g., example 19) relates to a method for Raman spectroscopy using a pixel arrangement. The pixel arrangement comprises at least one photo-sensitive pixel. The pixel arrangement further comprises a first charge storage and a second charge storage having respective charge levels configured to vary in response to interactions with charge carriers generated by the pixel arrangement. The method comprises emitting, for a plurality of measurement cycles in a measurement frame, respective excitation light to induce Raman scattering in a sample. The charge carriers are controlled by the pixel arrangement to interact with the charge storages in response to the excitation light based on at least one control signal. The method further comprises controlling the pixel arrangement via the at least one control signal in each measurement cycle to a) cause the charge carriers to vary the charge level of the first charge storage during an initial first phase of the respective measurement cycle for measuring background light; b) cause the charge carriers to vary the charge level of the second charge storage during a second phase of the respective measurement cycle following the first phase for measuring Raman light generated in response to the excitation light, the excitation light being emitted during the second phase of the respective measurement cycle; and c) drain the charge carriers from the at least one photo-sensitive pixel during a third phase of the respective measurement cycle extending from the end of the second phase through to the end of the respective measurement cycle. The pixel arrangement prevents interactions of the first and second charge storages with the charge carriers drained from the at least one photo-sensitive pixel during the third phase. The method comprises providing an output of the pixel arrangement for the measurement frame based on a voltage difference between the first and the second charge storage at the end of the measurement frame.

Another example (e.g., example 20) relates to use of the apparatus according to according to a previous example (e.g., one of the examples 1 to 17) or to any other example, the handheld or wearable device according to a previous example (e.g., example 18) or to any other example, or the method according to a previous example (e.g., example 19) or to any other example for at least one of determining a physiological property of an organism or foodstuff analysis.

The aspects and features described in relation to a particular one of the previous examples may also be combined with one or more of the further examples to replace an identical or similar feature of that further example or to additionally introduce the features into the further example.

It is further understood that the disclosure of several steps, processes, operations or functions disclosed in the description or claims shall not be construed to imply that these operations are necessarily dependent on the order described, unless explicitly stated in the individual case or necessary for technical reasons. Therefore, the previous description does not limit the execution of several steps or functions to a certain order. Furthermore, in further examples, a single step, function, process or operation may include and/or be broken up into several sub-steps, -functions, -processes or -operations.

If some aspects have been described in relation to a device or system, these aspects should also be understood as a description of the corresponding method. For example, a block, device or functional aspect of the device or system may correspond to a feature, such as a method step, of the corresponding method. Accordingly, aspects described in relation to a method shall also be understood as a description of a corresponding block, a corresponding element, a property or a functional feature of a corresponding device or a corresponding system.

The following claims are hereby incorporated in the detailed description, wherein each claim may stand on its own as a separate example. It should also be noted that although in the claims a dependent claim refers to a particular combination with one or more other claims, other examples may also include a combination of the dependent claim with the subject matter of any other dependent or independent claim. Such combinations are hereby explicitly proposed, unless it is stated in the individual case that a particular combination is not intended. Furthermore, features of a claim should also be included for any other independent claim, even if that claim is not directly defined as dependent on that other independent claim.

## Claims

1. An apparatus (100) for Raman spectroscopy, comprising:
an illumination source (110) configured to emit, for a plurality of measurement cycles in a measurement frame, respective excitation light (111) to induce Raman scattering in a sample; and
a pixel arrangement (120), the pixel arrangement (120) comprising at least one photo-sensitive pixel (121, 122), the pixel arrangement (120) comprising a first charge storage (123) and a second charge storage (124) having respective charge levels configured to vary in response to interactions with charge carriers generated by the pixel arrangement (120), the charge carriers being controlled by the pixel arrangement (120) to interact with the charge storages in response to the excitation light (111) based on at least one control signal (103), and the at least one control signal (103) being configured to control the pixel arrangement (120) in each measurement cycle to:
cause the charge carriers to vary the charge level of the first charge storage (123) during an initial first phase of the respective measurement cycle for measuring background light;
cause the charge carriers to vary the charge level of the second charge storage (124) during a second phase of the respective measurement cycle following the first phase for measuring Raman light generated in response to the excitation light (111), the excitation light (111) being emitted during the second phase of the respective measurement cycle; and
drain the charge carriers from the at least one photo-sensitive pixel (121, 122) during a third phase of the respective measurement cycle extending from the end of the second phase through to the end of the respective measurement cycle,
wherein the pixel arrangement (120) is configured to prevent interactions of the first and second charge storages (123, 124) with the charge carriers drained from the at least one photo-sensitive pixel (121, 122) during the third phase, and
wherein an output (125) of the pixel arrangement (120) for the measurement frame is based on a voltage difference between the first and the second charge storage (123, 124) at the end of the measurement frame.

2. The apparatus (100) of claim 1, wherein the pixel arrangement (120) is configured to retain the charge levels of the charge storages at the end of each measurement cycle and use them as starting charge levels for a following measurement cycle.

3. The apparatus (100) of claim 1 or claim 2, wherein the at least one photo-sensitive pixel (121, 122) is configured to separate the charge carriers generated in the photo-sensitive pixel (121, 122) during the measurement frame based on the at least one control signal (103).

4. The apparatus (100) of claim 1 or claim 2, wherein the pixel arrangement (120) comprises a first photo-sensitive pixel (121) configured to integrate incident light for varying the charge level of the first charge storage (123), and a second photo-sensitive pixel (122) configured to integrate the incident light for varying the charge level of the second charge storage (124), and wherein the at least one control signal (103) is configured to:
control the first photo-sensitive pixel (121) to integrate the incident light during the first phase of the respective measurement cycle such that the charge carriers vary the charge level of the first charge storage (123) during the first phase;
control the second photo-sensitive pixel (122) to integrate the incident light during the second phase of the respective measurement cycle such that the charge carriers vary the charge level of the second charge storage (124) during the second phase; and
control the first and the second photo-sensitive pixel (121, 122) to drain the charge carriers during the third phase of the respective measurement cycle.

5. The apparatus (100) of any one of claims 1 to 4, wherein, during a second measurement frame, the at least one control signal (103) is configured to control the pixel arrangement (120) to:
cause the charge carriers to vary the charge level of the second charge storage (124) during the first phase of the respective measurement cycle;
cause the charge carriers to vary the charge level of the first charge storage (123) during the second phase of the respective measurement cycle; and
drain the charge carriers from the at least one photo-sensitive pixel (121, 122) during the third phase of the respective measurement cycle, and
wherein an output (125) of the pixel arrangement (120) for the second measurement frame is based on a voltage difference between the charge storages at the end of the second measurement frame.

6. The apparatus (100) of any one of claims 1 to 5, wherein the first phase and the second phase are of equal length.

7. The apparatus (100) of any one of claims 1 to 6, wherein the illumination source (110) is configured to emit the respective excitation light (111) at the beginning of the respective second phase.

8. The apparatus (100) of any one of claims 1 to 7, further comprising processing circuitry configured to determine a characteristic of the sample based on the output (125) of the pixel arrangement (120) for the measurement frame.

9. The apparatus (100) of claim 8 when dependent on claim 5, wherein the processing circuitry (130) is configured to determine a characteristic of the sample based on an average determined based on the outputs (125) of the pixel arrangement (120) for the measurement frame and the second measurement frame.

10. The apparatus (100) of claim 8 or claim 9, wherein the sample is an organism and the characteristic of the sample is a physiological property of the organism.

11. The apparatus (100) of any one of claims 1 to 10, wherein the excitation light (111) exhibits a wavelength in the visible or near-infrared range.

12. The apparatus (100) of any one of claims 1 to 11, wherein a length of the third phase is set based on an expected duration of autofluorescence generated by the sample in response to the excitation light (111).

13. A handheld or wearable device (400) comprising an apparatus (100) for Raman spectroscopy according to any one of claims 1 to 12.

14. A method (500) for Raman spectroscopy using a pixel arrangement, the pixel arrangement comprising at least one photo-sensitive pixel, the pixel arrangement comprising a first charge storage and a second charge storage having respective charge levels configured to vary in response to interactions with charge carriers generated by the pixel arrangement, and the method (500) comprising:
emitting (502), for a plurality of measurement cycles in a measurement frame, respective excitation light to induce Raman scattering in a sample, the charge carriers being controlled by the pixel arrangement to interact with the charge storages in response to the excitation light based on at least one control signal;
controlling (504) the pixel arrangement via the at least one control signal in each measurement cycle to:
cause the charge carriers to vary the charge level of the first charge storage during an initial first phase of the respective measurement cycle for measuring background light;
cause the charge carriers to vary the charge level of the second charge storage during a second phase of the respective measurement cycle following the first phase for measuring Raman light generated in response to the excitation light, the excitation light being emitted during the second phase of the respective measurement cycle; and
drain the charge carriers from the at least one photo-sensitive pixel during a third phase of the respective measurement cycle extending from the end of the second phase through to the end of the respective measurement cycle, wherein the pixel arrangement prevents interactions of the first and second charge storages with the charge carriers drained from the at least one photo-sensitive pixel during the third phase; and
providing (506) an output of the pixel arrangement for the measurement frame based on a voltage difference between the first and the second charge storage at the end of the measurement frame.

15. Use of the apparatus (100) according to any one of claim 1 to 12, the handheld or wearable device (400) of claim 13 or the method (500) of claim 14 for determining a physiological property of an organism.
